# EUROPEAN PATENT APPLICATION

(11) **EP 4 613 764 A1**
(43) Date of publication of application: **10.09.2025**
(21) Application number: 24829014.0
(22) Date of filing: 24.05.2024
(51) Int. Cl.: C07K 14/78, A61Q 7/00, A61L 31/04, A61L 31/16, C12R 1/19

(54) **RECOMBINANT TYPE XVII COLLAGEN HAVING TRIPLE HELIX STRUCTURE AND USE THEREOF**

(30) Priority: 26.01.2024 CN 202410121405
(71) Applicant: SHENZHEN LIYING BIOTECHNOLOGY CO., LTD., Shenzhen, Guangdong 518107 (CN)
(72) Inventor: ZHANG, Xiaoyan, Shenzhen, Guangdong 518107 (CN); SONG, Xiaohui, Shenzhen, Guangdong 518107 (CN); FU, Juncheng, Shenzhen, Guangdong 518107 (CN); XU, Qiaoran, Shenzhen, Guangdong 518107 (CN); GE, Jianjing, Shenzhen, Guangdong 518107 (CN)
(74) Representative: J A Kemp LLP
(86) International application number: PCT/CN2024/095261
(87) International publication number: WO 2025/156530

(57) **Abstract**

The present disclosure provides a recombinant type XVII collagen with a triple helical structure and use thereof and belongs to the field of protein engineering technology. The recombinant type XVII collagen has the amino acid sequence formed by tandem repeats of n core units, wherein n is an integer of 1 or greater than 1, and the core unit has the amino acid sequence as set forth in SEQ ID NO: 1. The present disclosure also provides methods for constructing multiple expression systems and methods for preparing the recombinant type XVII collagen. The present disclosure also provides practical industrial applications based on the characteristics of the protein. The recombinant type XVII collagen provided in the present disclosure has high purity, good thermal stability, high biological activity, and a much smaller molecular weight than natural collagen, thus reducing obstacles for collagen to pass through the skin barrier to exert its biological function. It is experimentally shown that the recombinant type XVII collagen has multiple biological activities such as promoting cell migration.

## Description

### TECHNICAL FIELD

The present disclosure belongs to the field of protein engineering technology, and in particular relates to a recombinant type XVII collagen with a triple helical structure and use thereof.

### BACKGROUND

Type XVII collagen is found at an extremely trace amount in the body and is extremely difficult to obtain directly through extraction which is also costly. At present, in vitro mass production of the type XVII collagen is basically accomplished by means of recombination. The in vitro recombinant expression protein systems commonly used include E. coli, yeast, mammalian cells, etc.

Natural collagen has a unique triple helical structure, which is closely related to its stability and various biological activities such as promoting cell proliferation, adhesion, migration, etc. (Marion, 2010). Type XVII collagen is 1497 amino acids in length, and includes intracellular, transmembrane, and extracellular domains, which are further divided into 16 non-triple helical regions and 15 triple helical regions in alternative arrangment. Recombinant expression of type XVII collagen in vitro presents challenges due to its low yield and easy degradation. It is even more difficult to assemble the expressed type XVII collagen into a natural conformation of alternative non-triple helix and triple helix.

In Patent Publication No. CN110845603A, a truncated sequence of the C15 helical region and about 130 amino acids at the C-terminus of type XVII collagen were selected for expression and verification, resulting in good yield and no post-translational modification activity by using the E. coli expression system. In Patent Publication No. CN116640205A, a Pichia pastoris expression system was used, and truncated sequences of non-helical region NC16 and C15 helical region and the C-terminus of type XVII collagen were selected for splicing and combination. In Patent Publication No. CN116751282A, a Saccharomyces cerevisiae expression system was used, and the truncated sequence of the C15 helical region and the C1 helical region were selected for splicing and combination. However, none of the above technologies showed that the natural triple helical structure of collagen could be formed.

Collagen has natural micelle structure and large molecular weight, making it difficult to penetrate through the skin barrier into the dermis to exert its effects when used as an ingredient in skin care products.

In summary, the prior art has the following problems: 1) the recombinant collagen is not subjected to post-translational modification due to the limitation by the conventional prokaryotic expression system; 2) no higher-order triple helical conformation is formed; 3) low biological activity; 4) poor stability; and 5) the natural collagen has a large molecular weight, making it hard to penetrate through the skin, etc.

### SUMMARY

In view of this, a first objective of the present disclosure is to provide a recombinant type XVII collagen with a triple helical structure. The recombinant collagen provided in the present disclosure has high purity, good thermal stability, high biological activity, and a molecular weight that is significantly smaller than a natural full-length collagen. It is experimentally shown that the recombinant type XVII collagen has multiple biological activities such as promoting cell migration.

In the present disclosure, the recombinant type XVII collagen has an amino acid sequence formed by tandem repeats of n core units, wherein n is an integer of 1 or greater than 1, and the core unit has the amino acid sequence as set forth in SEQ ID NO: 1.

Preferably, n is any numerical value selected from 3, 4, 5, 6, 7, or 8. Most preferably, n is selected from 8.

The tandem repeats may be formed by head-to-tail tandem connection of core units, or by tandem connection of core units via a linker. The linker preferably contains glycine (Gly) and/or serine (Ser), and the linker preferably contains 1 to 5 amino acids.

The present disclosure provides a gene encoding said recombinant type XVII collagen.

The encoding comprises transcripting DNA molecule to form a RNA product, and then translating to produce the protein; or transcripting the DNA molecular to provide a RNA product, process the RNA product to provide the processed RNA product, and then translating to produce the protein.

The present disclosure provides a recombinant vector for expressing said recombinant type XVII collagen, including said gene.

The vector includes any nucleic acid molecule (e.g., plasmids, cosmids, viruses, autonomously replicating polynucleotide molecules, bacteriophages, or linear or circular single-stranded or double-stranded DNA or RNA nucleic acid molecules) derived from any source and capable of being integrated into a genome or autonomous replication. The vector contains one or more nucleic acid molecules that have been operably linked. The vector may include, for example, one or more selectable markers, one or more replication origins (e.g., prokaryotic and eukaryotic origins), at least one multiple cloning site, and/or elements that promote stable integration of the construct into a genome of a host cell. Preferably, the vector includes, but is not limited to, pET28a, pCDFDuet-1, pPICZαA, and pPIC9k.

In the present disclosure, the recombinant human type III collagen gene alone may be cloned into the vector, or the recombinant human type III collagen gene may be cloned into the same vector together with proline hydroxylase coding gene and/or lysine hydroxylase coding gene.

The present disclosure provides a recombinant cell expressing the recombinant type XVII collagen, wherein the recombinant cell is transformed with said recombinant vector.

The recombinant cell includes, but is not limited to, E. coli and Pichia pastoris.

A second objective of the present disclosure is to provide a method for expressing the recombinant type XVII collagen.

In a preferred embodiment, the recombinant type XVII collagen is expressed in E. coli, preferably in E. coli BL21 (DE3), including:
1) ligating said gene to a vector pET28a to obtain a recombinant vector;
2) transforming E. coli BL21 (DE3) with the recombinant vector to obtain a recombinant cell;
3) culturing the recombinant cell, followed by centrifugation and collecting a supernatant; and
4) separating, purifying and obtaining the recombinant type XVII collagen from the supernatant.

More preferably, the method further comprises constructing gene segments encoding hydroxylases L593 and L230 into an expression vector pCDFDuet-1 to obtain a recombinant vector pCDFDuet-1-L593-L230, and transforming E. coli BL21 (DE3) with the recombinant vector.

More preferably, the culturing in 3) includes adding ascorbic acid at a final concentration of 10 mg/ml, FeSO₄ at a final concentration of 1 mM, and IPTG at a final concentration of 1 mM to induce the expression of the target protein, which can further improve the thermal stability of the target protein.

In another preferred embodiment, the recombinant type XVII collagen is expressed in E. coli, preferably in Pichia pastoris GS115, comprising:
1) ligating said gene to a vector pPICZαA to obtain a recombinant vector;
2) transforming Pichia pastoris GS115 with the recombinant vector to obtain a recombinant cell;
3) culturing the recombinant cell followed by centrifugation, and collecting a supernatant; and
4) separating and obtaining the recombinant type XVII collagen from the supernatant.

More preferably, the method further comprises constructing gene segment encoding hydroxylase BaP4H into the expression vector pPIC9k to obtain a recombinant vector pPIC9k-BaP4H, and transforming Pichia pastoris GS115 with the recombinant vector.

In addition to being a key factor in skin aging and wound repair, type XVII collagen also plays an important role in maintaining hair follicle stem cells to prevent hair loss and gray hair. Col17A1 is highly expressed in hair follicle stem cells (HFSCs), and Col17A1 is essential to maintain hair follicle stem cells and melanocyte stem cells (MSCs) (Shintaro Tanimura, 2011). Due to its good biocompatibility and biodegradability, collagen has been widely studied and applied in the field of biomaterials, such as tissue engineering scaffolds, absorbable sutures, etc.

Thus, a third objective of the present disclosure is to provide said recombinant type XVII collagen, and a recombinant type XVII collagen obtained by said preparation method, for use in:
i) promoting cell proliferation;
ii) promoting cell migration;
iii) preparing a cosmetic; or
iv) preparing a medical material.

Promoting cell proliferation can refer to its use as a basic component of a culture medium to promote cell proliferation, including the use in the culture and preservation of hair follicle stem cells or hair follicle tissues in vitro.

Promoting cell migration can refer to its use as a basic component of a culture medium to promote cell migration, including the use in the culture and preservation of hair follicle stem cells or hair follicle tissues in vitro.

The cosmetic includes, but is not limited to, skin care lotion, skin care cream, essence, facial mask, scalp essence, and shampoo.

The medical material includes, but is not limited to, filling materials, repair materials, implants, tissue engineering scaffolds, hemostatic agents, and drug release carriers, wherein the repair materials include, but are not limited to, bone repair materials, wound dressings, and sutures.

The present disclosure also provides a composition including a cosmetically or pharmaceutically effective amount of at least one protein as described above and at least one excipient or cosmetically or pharmaceutically acceptable adjuvant. In some embodiments, the composition is in a dosage form including but not limited to creams, lotions, aqueous solutions, gels, oils, powders, muds, patches, films or freeze-dried products. Further, in order to promote the transdermal absorption of collagen molecules, patch products can be prepared using solid carriers such as non-woven fabrics. The patch products are applied on a face to prolong the time for the collagen solution acting on the skin surface. The recombinant type XVII collagen of the present disclosure can be used after being prepared in combination with a bioactive ingredient, such as recombinant or natural type I collagen, type II collagen, type III collagen, type IV collagen, type V collagen, fibronectin, human epidermal growth factor, etc.,, or can also be used after being prepared separately from and then mixed with the above-mentioned bioactive ingredients.

Compared with the prior art, the present disclosure has the following beneficial effects:
1) The recombinant type XVII collagen of the present disclosure has a theoretical molecular weight of about 33 kDa, significantly smaller than the natural full-length collagen (150 kDa), thus reducing obstacles for collagen to pass through the skin barrier and exert its biological function, and providing the effect of promoting cell migration;
2) The recombinant type XVII collagen of the present disclosure has a higher-order triple helical structure, which is a stable molecular structure, enabling its thermal stability to be maintained at 40°C;
3) The recombinant type XVII collagen of the present disclosure has a sequence that is a novel recombinant type XVII collagen sequence obtained by optimizing and selecting a segment with high biological activity and stable high-order structure based on the original amino acid sequence of human type XVII collagen, and has the characteristics of good biocompatibility, etc.; and
4) The recombinant type XVII collagen of the present disclosure has excellent cell migration promoting activity.

### BRIEF DESCRIPTION OF THE DRAWINGS

FIG. 1 is an SDS-PAGE diagram of the recombinant type XVII collagen LY1701 (n=3) of the present disclosure expressed from an E. coli expression system.
FIG. 2 is an SDS-PAGE analysis of the recombinant type XVII collagen LY1701 (n=4) of the present disclosure expressed from an E. coli expression system.
FIG. 3 is an SDS-PAGE analysis of the recombinant type XVII collagen LY1701 (n=5) of the present disclosure expressed from an E. coli expression system.
FIG. 4 is an SDS-PAGE analysis of the recombinant type XVII collagen LY1701 (n=6) of the present disclosure expressed from an E. coli expression system.
FIG. 5 is an SDS-PAGE analysis of the recombinant type XVII collagen LY1701 (n=7) of the present disclosure expressed from an E. coli expression system.
FIG. 6 is an SDS-PAGE analysis of the recombinant type XVII collagen LY1701 (n=8) of the present disclosure expressed from an E. coli expression system.
FIG. 7 is an SDS-PAGE analysis of the recombinant type XVII collagen LY1701 (n=8) of the present disclosure expressed from a Pichia pastoris expression system.
FIG. 8 is a circular dichroism spectrum of the recombinant type XVII collagen LY1701 (n=3, 4, 5, 6, 7, or 8) of the present disclosure at room temperature.
FIG. 9 is a circular dichroism spectrum of the recombinant type XVII collagen LY1701 (n=3, 4, 5, 6, 7, or 8) of the present disclosure at various temperatures.
FIG. 10 shows photographs showing the cell scratch assay results of the recombinant type XVII collagen LY1701 (n=8) of the present disclosure.
FIG. 11 is a graph showing the cell scratch recovery area analysis of the recombinant type XVII collagen LY1701 (n=8) of the present disclosure.

### DETAILED DESCRIPTION

In the present disclosure, the genes can be synthesized by a biotechnology company. In the present disclosure, the preparation methods of the recombinant vectors and recombinant cells are not particularly limited, and can be the conventional preparation methods of the recombinant vectors and recombinant cells in the art.

In the present disclosure, the separation and purification methods are not particularly limited, and the conventional separation and purification methods for proteins in the art may be used. The preferred technical schemes are described in the Examples.

In the present disclosure, the structure of collagen is characterized by circular dichroism (CD) commonly used in the art. CD is a spectroscopic method used to determine the structure of compounds with chiral structures that can produce left-right optical rotation differential absorption, and mainly used to determine the asymmetry of molecular structures. Since biomacromolecules generally contain a chiral group and structure, CD is often used to measure and observe the structure and changes in conformation of biomacromolecules. The triple helical structure of collagen generally has CD characteristics that there is a positive absorption peak near 221 nm and a negative absorption peak near 195 nm (Industry standard Y Y/T 1849-2022), and the position of the absorption peak will shift with changes in the amino acid sequence and length thereof. The thermal stability of collagen includes a thermal shrinkage temperature (Ts) of collagen fibers and a thermal denaturation temperature (Td) of collagen. The thermal shrinkage temperature of collagen fibers refers to a temperature at which the collagen fibers shrink axially and shorten by about 5% of their original length when heated. The thermal denaturation temperature of collagen refers to a temperature at which the triple helix unwinds to the extent of 50% to form single strands when the collagen is heated in a medium. Therefore, CD spectra can be used to study the helical structure of collagen and its thermal denaturation process.

In the following Examples, LY1701 represents a series of proteins formed by tandem repeats of core units (SEQ ID NO: 1), wherein the series of proteins includes core units repeated 3, 4, 5, 6, 7, or 8 times, respectively.

The technical solutions provided in the present disclosure are described in detail below in conjunction with the Examples, but which should not be construed as limiting the protection scope of the present disclosure.

### Example 1. Expression (E. coli expression system), Separation, and Purification of Recombinant Type XVII Collagen LY1701 of Present Disclosure

### 1. Synthesis of LY1701 sequence

Gene segments for encoding the amino acid sequences of the recombinant type XVII collagen LY1701 of the present disclosure (SEQ ID NO: 2, SEQ ID NO: 3, SEQ ID NO: 4, SEQ ID NO: 5, SEQ ID NO: 6, SEQ ID NO: 7) were synthesized by GenScript Biotech Co., Ltd, and constructed into a pET28a E. coli expression vector. Gene segments for encoding hydroxylases L593 and L230 were synthesized by GenScript Biotech Co., Ltd, and constructed into an expression vector pCDFDuet-1, downstream of two promoters.

### 2. Construction of E. coli expression system

### 2.1 Transformation

The two plasmids, type XVII collagen plasmid pET28a-LY1701 (Kanamycin resistance) and hydroxylase plasmid pCDFDuet-1-L593-L230 (Streptomycin resistance), were co-transformed into competent cells BL21 (DE3) (AlpalifeBio, Shenzhen). The cells were placed on ice for 30 min, heat-shocked in a 42°C water bath for 45 s, and immediately placed on ice for 2 to 3 min, and then 900 µl SOC or LB medium (without antibiotics) was added. The cells were incubated in a shaker at 37°C and at a speed of 200 to 250 rpm for 1 h. At the same time, LB solid medium plates with both kanamycin and streptomycin resistance (10 g/L peptone, 5 g/L yeast extract, 10 g/L sodium chloride, 15 g/L agar, 100 µg/ml kanamycin antibiotic) were preheated in a 37°C incubator. After incubation in the shaker, the cells were centrifuged at 5000 rpm (2400 x g) for 5 min, with 900 µl of supernatant discarded. The bacteria were resuspended with the remaining culture medium and spread evenly on the preheated double-resistance plate with a sterile spreader. The plate was incubated invertedly in the incubator at 37°C for 12 to 16 h until the colonies grew to be clearly visible.

2.2 The plates with colonies were taken out, and monoclonal colonies were picked out and inoculated into 10 ml LB liquid culture medium (containing kanamycin and streptomycin antibiotics). The medium was cultured at 37°C and 220 rpm overnight, and then added into 1 L LB culture medium (containing kanamycin and streptomycin antibiotics) at a ratio of 1:100. After cultured at 37°C and 220 rpm for 3 to 5 h, the OD value was monitored. Once the OD₆₀₀ value reached 1.0 to 1.2, ascorbic acid (Sangon Biotech, Shanghai) at a final concentration of 10 mg/ml, FeSO₄ (Sangon Biotech, Shanghai) at a final concentration of 1 mM and IPTG (Biosharp) at a final concentration of 1 mM were added to induce expression at 16°C and 180 rpm for 18 to 24 h. The bacteria were collected by centrifugation for the next step of purification or for cryopreservation at -20°C or lower.

### 3. Purification of Protein

3.1 After being sampled and subjected to gel electrophoresis for later use, the induced bacterial solution was centrifuged at 8000 rpm for 20 min to collect the bacteria.

### 3.2 Preparation of Ni-NTA (Sangon Biotech) purification buffer:

Lysis buffer: 50 mM Tris-HCl, 500 mM NaCl, 10 mM imidazole, and 5% glycerol, pH 7.0;
Wash buffer: 50 mM Tris-HCl, 500 mM NaCl, 20 mM imidazole, and 5% glycerol, pH 7.0;
Elution buffer: 50mM Tris-HCl, 500mM NaCl, 300mM imidazole, and 5% glycerol, pH 7.0.

### 3.3 Preparation of Lysis buffer

Ingredients of Lysis buffer: PMSF (MIKX) at a final concentration of 1mM, protease inhibitor (MCE), super nuclease (Beyotime), and MgCl₂ (Sangon Biotech) at a final concentration of 1mM.

Depending on the weight of the bacteria, a volume of Lysis buffer was added at a ratio of 1:10, and the bacteria were resuspended using a homogenizer, and mixed well by pipetting.

### 3.4 Affinity purification

A high-pressure homogenizer (Duoning Biotech) was turned on in advance for precooling. The bacterial solution was added and homogenized under 700 to 800 bar for 3 times, and then the disrupted bacterial solution was collected. The bacterial solution was centrifuged in a high-speed refrigerated centrifuge (Beckman) at 4°C and 20000 rpm for 1h. After centrifugation, the supernatant was transferred to a 50ml fresh centrifuge tube and filtered with a 0.45 µm filter. A Ni-NTA gravity column (1ml Ni-NTA) was washed with 5 column volumes of filtered pure water and with 3 column volumes of Lysis buffer, and then loaded with the sample using a peristaltic pump in which the channels had been washed with pure water in large quantities and rinsed with Lysis buffer. Then, the Ni-NTA gravity column was loaded with the filtered supernatant using the peristaltic pump, and the flow-through solution was collected. The Ni-NTA was washed with 100ml of wash buffer and the wash solution was collected. The target protein was eluted by adding 1 ml of elution buffer each time and incubating for 5 min, and the eluate was collected.

### 3.5 Purification by Ion Exchange Chromatography

The target protein was dialyzed into buffer A (20 mM PB, PH=7.0), and the protein sample was filtered through a 0.22 µm, 13 mm filter membrane, and subjected to cation exchange using AKTA pure system (5ml SP column), and then linear elution with Buffer B (20 mM PB and 1M NaCl, PH=7.0) to obtain the target protein with higher purity. The molecular weight and purity of the protein were verified by SDS-PAGE electrophoresis. The protein sample was mixed with the protein loading buffer (with DTT), placed in a metal bath, boiled at 95°C for 10 min, and then loaded for gel electrophoresis. The protein concentration was determined by BCA method to estimate the protein yield. The protein was concentrated or lyophilized according to its purity and stored.

### Results

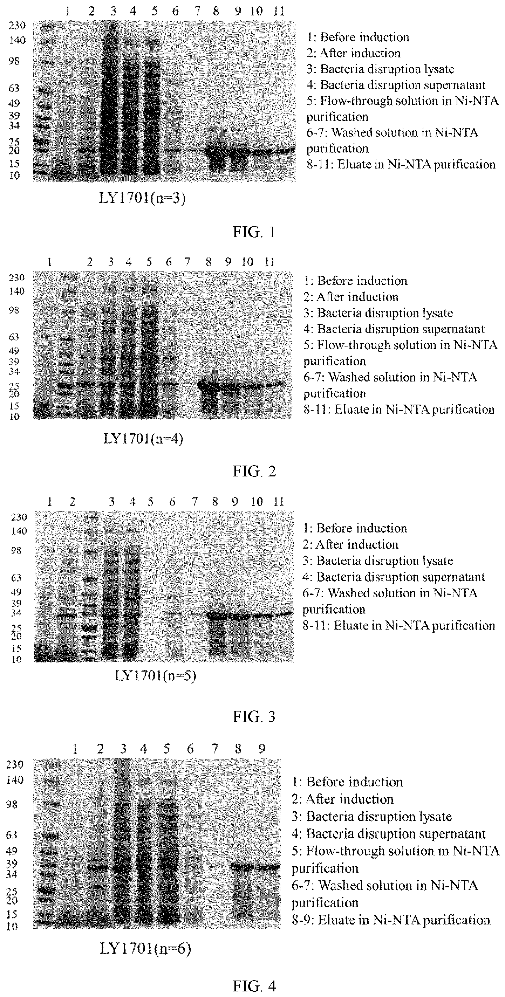

FIGS. 1 to 6 are SDS-PAGE electrophoresis diagrams of the recombinant type XVII collagen of the present disclosure after expression in E. coli and purification.

### Example 2. Expression (in Pichia Pastoris Expression System), Separation and Purification of Recombinant Type XVII Collagen LY1701 of Present Disclosure

### 1. Synthesis of LY1701 sequence

A gene segment encoding an amino acid sequence of the recombinant type XVII collagen LY1701 of the present disclosure (SEQ ID NO: 7) was synthesized by GenScript Biotech Co., Ltd, and constructed into a pPICZalphaAPichia pastoris expression vector. Agene segment for encoding hydroxylase BaP4H was synthesized by GenScript Biotech Co., Ltd, and constructed into a vector pPIC9k.

### 2. Construction of Pichia pastoris expression system

### 2.1 Electroporation

The type XVII collagen plasmid pPICZalphaA- LY1701 was linearized at the SacI site by PCR amplification, and then the PCR fragment was recovered (Thermo GeneJET PCR Purification Kit). Competent cells GS115 were thawed on ice and, added with 5 to 10 µg linearized plasmid, and incubated on ice for 15 min. The plasmid and competent cells were added into a pre-cooled electroporation cuvette. Electroporation was carried out at voltage of 2000 V, resistance of 200 Ω, capacitance of 25 µF, electroporation cuvette diameter of 0.2 mm, and time constant of 5 ms. 1ml pre-cooled sorbitol was added immediately after electroporation, and the mixture was transferred to a 1.5 ml sterile centrifuge tube and activated at 30°C and 230rpm for 2 h. The mixture was centrifuged at 5000 g for 1 min and 300 µl supernatant was discarded. The cells were resuspended and spread on a YPDSZ solid plate (tryptone 20 g/L, yeast extract fermentation 10 g/L, D-sorbitol 182.1 g/L, 20% glucose, and 100µg/ml bleomycin), and cultured at 30°C and 230 rpm for 48 h.

2.2 The plates with colonies were taken out, and monoclonal colonies were picked out and replicated on a YPD solid medium with high concentration (400µg/ml or 800µg/ml) of bleomycin for screening.

2.3 The plates with colonies were taken out, and monoclonal colonies were picked out and inoculated into 50 ml YPD liquid culture medium (containing 100µg/ml bleomycin). The seed liquid was cultured at 30°C and 230 rpm overnight. The overnight cultured seed liquid was inoculated into 1 L BMGY medium (tryptone 20 g/L, yeast extract fermentation 10g/L, potassium dihydrogen phosphate (KH₂PO₄) 11.73g/ L, potassium phosphate dibasic (K₂HPO₄) 2.4g /L, glycerol 10g/L, YNB 134g/L and 1 x biotin) at a ratio of 1:50 for amplification, and cultured at 30°C and 230 rpm for 48 h. The seed liquid cultured in BMGY was centrifuged at 3000 rpm for 5 min, and the supernatant was discarded. The cells were resuspended in sterile water and centrifuged again at 3000 rpm for 5 min, and the supernatant was discarded. The cells was resuspended in 1 L BMMY medium (without methanol) and induced by adding 1% methanol, with 1% methanol further added every 24 h. After induction for 72 h, the induced liquid was centrifugation and the supernatant was collected and purified.

### 2.4 Purification by Ion Exchange Chromatography

The target protein was dialyzed into buffer A (20 mM PB, PH=7.0). The protein sample was filtered through a filter membrane with a pore size of 0.22 µm and a diameter of 13 mm, and subjected to cation exchange by using AKTA pure system (5 ml SP column) and linear elution with buffer B (20 mM PB and 1M NaCl, PH=7.0) to obtain the target protein with higher purity. The molecular weight and purity of the protein were verified by SDS-PAGE electrophoresis. The protein sample was mixed with the protein loading buffer (with DTT), placed in a metal bath, heated to 95°C for 10min, and centrifuged at 1500 rpm for 1 min. Then the supernatant was sampled for SDS-PAGE analysis. The protein concentration was determined by BCA method to estimate the protein yield. The protein was concentrated or lyophilized according to its purity and stored.

### 2.5 Preparation of Yeast Competent Cells Containing LY1701 Expression Plasmid

LY1701 monoclonal colonies with high expression yield were picked out and cultured in 5 ml YPD medium (Bleomycin resistance) at 30°C and 230 rpm overnight. The culture was inoculated in 500 ml fresh YPD medium (Bleomycin resistance) at a ratio of 1:200 and cultured overnight until OD₆₀₀ reached between 1.3 and 1.5. The cells were collected by centrifugation at 4°C and 1500g for 5 min, washed with 500 ml pre-cooled sterile water and resuspended. The resuspended cells was collected again by centrifugation at 4°C and 1500g for 5 min, and washed 3 times with pre-cooled sterile water. The cells were washed with 20 ml of 1M sorbitol and resuspended. The supernatant was discarded. After repeatedly washing 2 or 3 times, the cells were collected by centrifugation at 4°C and 1500g for 5 min, resuspended with 500µl of 1M sorbitol, and stored in a -80°C refrigerator at 100µl per tube.

### 2.6 BaP4H-pPIC9k linearization and electroporation

Primers were design for linearization at the SalI site of pPIC9k by PCR, and the PCR fragment (Thermo GeneJET PCR Purification Kit) was recovered. The yeast competent cells containing the LY1701 expression plasmid obtained in Example 2.4 were thawed on ice, added with 5 to 10 µg linearized plasmid, and incubated on ice for 15min. The plasmid and competent cells were added to a pre-cooled electroporation cuvette. Electroporation was carried out at voltage of 2000 V, resistance of 200 Ω, capacitance of 25 µF, electroporation cuvette diameter of 0.2 mm, and time constant of 5 ms. 400µl pre-cooled sorbitol was added immediately after electroporation, and the mixture was transferred to a 1.5 ml sterile centrifuge tube and activated at 30°C and 230 rpm for 2 h. The mixture was centrifuged at 5000 g for 1 min and 300µl supernatant was discarded. The cells were resuspended and spread on a YPDSZ solid plate (tryptone 20 g/L, yeast extract fermentation 10 g/L, 20% glucose, and 4 mg/ml G418), and cultured at 30°C and 230 rpm for 48 h.

### 2.7 Induction of expression

A single colony which grew well on the resistance plate was picked out and inoculated into 1 L BMGY medium, and cultured at 30°C and 230 rpm for 48 h. The culture was centrifuged at 3000 rpm for 5 min, and the supernatant was discarded. The cells were resuspended in 500ml sterile water and centrifuged again at 3000 rpm for 5 min, and the supernatant was discarded. The cells were resuspended in 1 L BMMY medium (without methanol) and the mixture was transferred to a conical flask. Then 1% methanol was added, with 1% methanol further added every 24 h. After induction for 72 h, centrifugation was carried out and the supernatant was collected and purified.

### 2.8 Purification by Ion Exchange Chromatography

The target protein was dialyzed into buffer A (20mM PB, PH=7.0), and the protein sample was filtered through a filter membrane with a pore size of 0.22µm and a diameter of 13mm and subjected to cation exchange by using AKTA pure (5ml SP column) and linear elution with Buffer B (20mM PB and 1M NaCl, PH=7.0) to obtain the target protein with higher purity. The molecular weight and purity of the protein were verified by SDS-PAGE electrophoresis. The protein sample was mixed with the protein loading buffer (with DTT), placed in a metal bath, heated to 95°C for 10min, and centrifuged at 1500 rpm. Then the supernatant was sampled for SDS-PAGE analysis. The protein concentration was determined by BCA method to estimate the protein yield. The protein was concentrated or lyophilized according to its purity and stored.

### Results

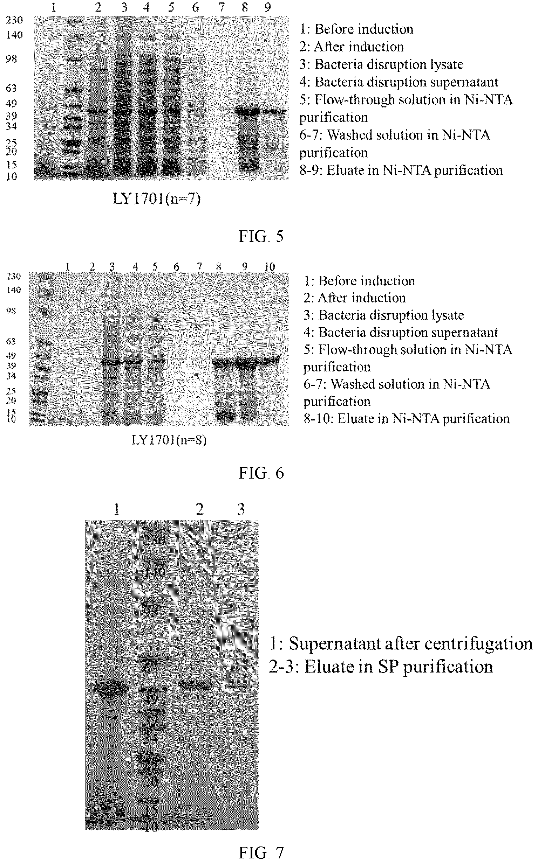

FIG. 7 is a SDS-PAGE electrophoresis diagram of the recombinant type XVII collagen of the present disclosure after expression in Pichia pastoris and purification. The recombinant type XVII collagen has a molecular weight of 49 KDa (theoretical value is 33.75 KDa).

### Example 3. Determination of triple helical conformation of Type XVII Collagen by Circular Dichroism (CD)

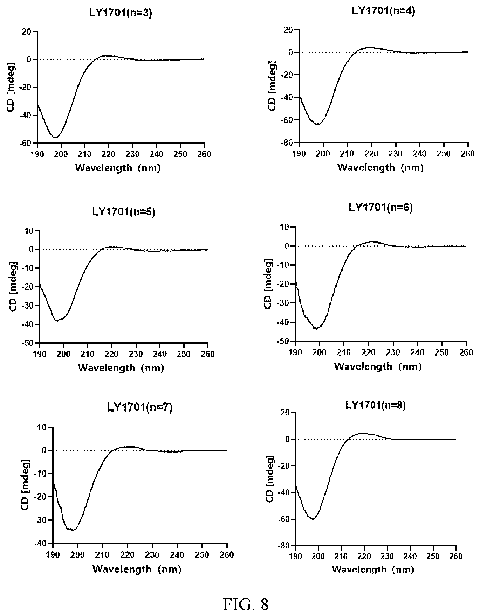

The type XVII collagen freeze-dried powder LY1701 prepared in Example 1 was dissolved in 20 mM PB buffer (PH7.4) to a concentration of 0.5 mg/ml. The sample was further diluted to a concentration of 0.01 mg/ml in a volume of 3 ml. The sample to be tested was transferred to a 10mm × 10mm sample cuvette in a circular dichroism spectrometer with the scanning wavelength range set between 190nm and 260nm, the scanning speed at 100nm/min, and the scanning temperature at room temperature. The CD spectra were averaged from three scans. As shown in FIG. 8, the recombinant type XVII collagen of the present disclosure has a maximum characteristic positive peak at 221nm, and a negative peak appearing at less than 200nm. According to the circular dichroism characteristics of the known collagen triple helical structure, it is determined that the collagen sample has a triple helical structure.

### Example 4. Determination of Thermal Stability of type XVII collagen by Circular Dichroism (CD)

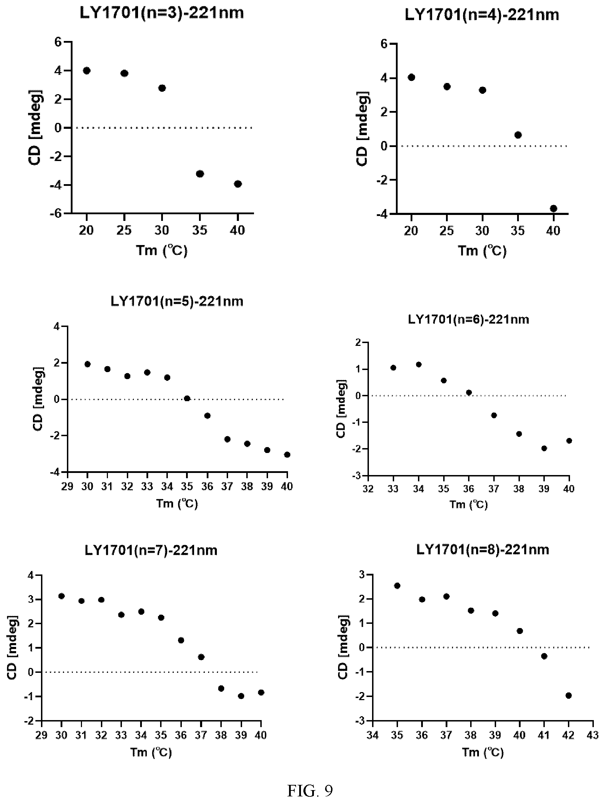

The collagen samples were diluted with reference to the method described in Example 3, and tested for the thermal stability of the protein by real-time heating at a rate of 1°C/min. The CD spectra were averaged from three scans. The data were analyzed and calculated on the software. The relationship between the molar ellipticity and temperature of the recombinant collagen was determined at 221nm.The results are shown in FIG. 9, and based on the disappearance of the characteristic positive peak at 221nm, the preferred thermal stability of LY1701 can be maintained at 40°C.

### Example 5. Determination of Cell Migration Activity of Type XVII Collagen by Cell Scratch Assay

Cell scratch assay is a commonly used method to detect the cell migration activity of collagen. A higher migration rate indicates better biological activity of the collagen. The specific steps are as follows:
1. Mouse 3T3 cells (Haixing Biotech) were resuscitated and passaged 3 days in advance to maintain a good state and viability. Once the cell viability reached more than 98%, the cells were seeded in a 6-well plate at a density of 5×10⁵ cells/ml and a plating volume of 2 ml. The cells were incubated in an incubator at 37°C, 5% CO₂ for 24h. Once the cells reach >90% confluency in the 6-well plate, the next scratch experiment was performed.
2. The lyophilized type XVII collagen powder LY1701 (SEQ ID NO: 7) to be tested for experimental group, and BSA and commercially available recombinant collagen products (competitors 1 and 2) for control groups were dissolved in 20mM PB buffer (PH7.4), respectively. These proteins were measured for the concentration after dissolution and diluted to a concentration of 0.5mg/ml.
3. A ruler that has been exposed to ultraviolet light for 30 minutes and a black marker were used to draw parallel horizontal lines with an interval between 0.5 cm and 1 cm on the bottom of the 6-well plate. At least 3 lines were drawn for each well. After the horizontal lines were drawn, the plate was marked with respective marks for subsequent observation.
4. A scratch perpendicular to the lines on the bottom of the 6-well plate was made in the confluent monolayer of cells using a cell scraper or 300ul pipette tip perpendicularly to the surface of the 6-well plate, with the width of the scratch kept consistent as much as possible during scratching. The 6-well plate was washed 3 times with PBS to remove free cells and cell debris caused by scratches. 2 ml of DMEM serum-free medium and the prepared protein solution were added to the wells so that the final concentration of the protein was 50 µg/ml. The plate was placed in a incubator at 37°C and 5% CO₂and stood for culture.
5. The cell scratches were observed and photographed under a microscope at 0 h and 12 h. The cell migration photographs were processed using Image J software to obtain the area of initial scratch and the blank area after cell migration. The migration rate was calculated. Migration rate = (Area of initial scratch - Blank area after cell migration) / Area of initial scratch * 100%.

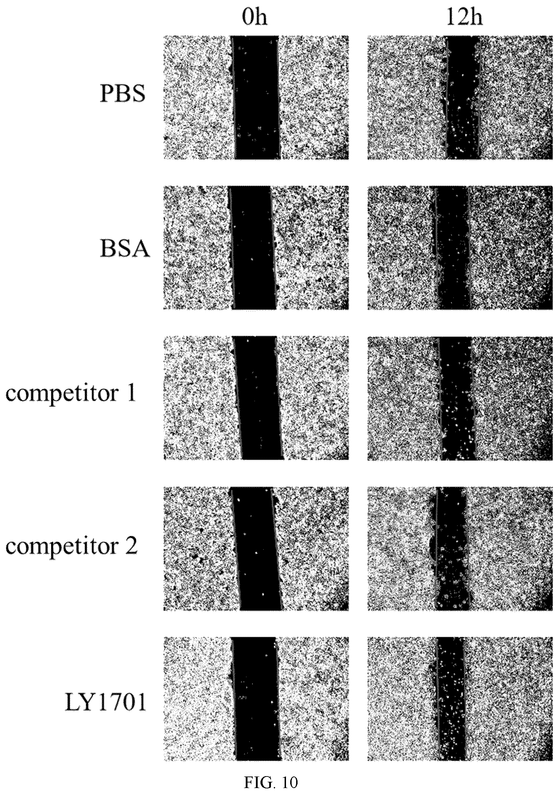

The results are shown in FIG. 10. After only 12 hours post treatment, the recovering area of cell scratch by the recombinant type XVII collagen LY1701 cells of the present disclosure was significantly reduced compared with the control groups, and the cell aggregation phenomenon in the blank area was more significant than that in other groups.

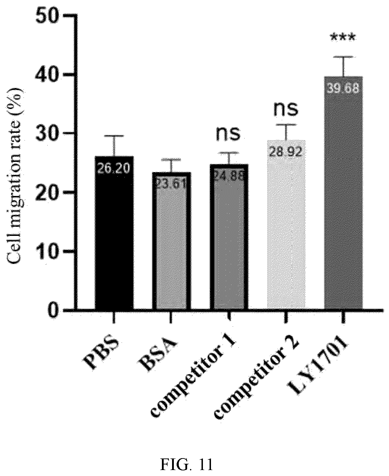

As shown in FIG. 11, data analysis shows that the recovering area of cell scratch was 23.61% for BSA -treated group and 39.68 % for the recombinant type XVII collagen LY1701, while the recovering cell scratch area for the competitor 1 collagen-treated group was 24.88 %, and 28.92 % for the competitor 2 collagen-treated group.

It can be seen that the type XVII collagen LY1701 of the present disclosure has an excellent effect of promoting cell migration.

The above is merely preferred embodiments of the present disclosure. It should be pointed out that several improvements and modifications can be made for ordinary technicians in this technical field without departing from the principle of the present disclosure. These improvements and modifications should also be regarded as within the scope of protection of the present disclosure.

## Claims

1. A recombinant type XVII collagen having the amino acid sequence formed by tandem repeats of n core units, wherein n is an integer of 1 or greater than 1, and the core unit has the amino acid sequence as set forth in SEQ ID NO: 1.

2. The recombinant type XVII collagen according to claim 1, wherein n is any numerical value selected from 3, 4, 5, 6, 7, or 8.

3. A gene encoding the recombinant type XVII collagen according to claim 1 or 2.

4. A recombinant vector for expressing the recombinant type XVII collagen according to claim 1 or 2, comprising the gene according to claim 3.

5. A recombinant cell expressing the recombinant type XVII collagen according to claim 1, wherein the recombinant cell is transformed with the recombinant vector according to claim 4.

6. A method for preparing the recombinant type XVII collagen according to claim 1, comprising:
1) recombining the gene according to claim 3 into a vector pET28a to obtain a recombinant vector;
2) transforming E. coli BL21 (DE3) with the recombinant vector to obtain a recombinant cell;
3) culturing the recombinant cell followed by centrifugation, and collecting a supernatant; and
4) separating the recombinant type XVII collagen from the supernatant.

7. The method according to claim 6, further comprising constructing gene segments encoding hydroxylases L593 and L230 into an expression vector pCDFDuet-1 to obtain a recombinant vector pCDFDuet-1-L593-L230, and transforming E. coli BL21 (DE3) with the recombinant vector.

8. The method according to claim 6, wherein the culturing in 3) comprises adding ascorbic acid at a final concentration of 10 mg/ml, FeSO₄ at a final concentration of 1 mM, and IPTG at a final concentration of 1 mM to induce the expression of the target protein.

9. A method for preparing the recombinant type XVII collagen according to claim 1, comprising:
1) recombining the gene according to claim 3 into a vector pPICZαA to obtain a recombinant vector;
2) transforming Pichia pastoris GS115 with the recombinant vector to obtain a recombinant cell;
3) culturing the recombinant cell followed by centrifugation, and collecting a supernatant; and
4) separating the recombinant type XVII collagen from the supernatant.

10. The method according to claim 9, further comprising constructing gene segment encoding hydroxylase BaP4H into an expression vector pPIC9k to obtain a recombinant vector pPIC9k-BaP4H, and transforming Pichia pastoris GS115 with the recombinant vector.

11. A recombinant type XVII collagen according to claim 1, or a recombinant type XVII collagen obtained by a method according to claim 6 or 9, for use in:
i) promoting cell proliferation;
ii) promoting cell migration;
iii) preparing a cosmetic; or
iv) preparing a medical material.
